# EUROPÄISCHE PATENTSCHRIFT

(11) **EP 0 554 579 B1**
(45) Veröffentlichungstag und Bekanntmachung des Hinweises auf die Patenterteilung: **27.03.1996**
(21) Anmeldenummer: 92200294.4
(22) Anmeldetag: 03.02.1992
(51) Int. Cl.: A61F 2/06

(54) **Katheter mit einer Gefässstütze**
Catheter with vessel support
Cathéter avec un support vasculaire

(43) Veröffentlichungstag der Anmeldung: 11.08.1993
(73) Patentinhaber: SCHNEIDER (EUROPE) AG, CH-8180 Bülach (CH)
(72) Erfinder: Imbert, Christian, CH-1290 Versoix (CH); Hofmann, Eugen, CH-8064 Zürich (CH); Gianotti, Marc, CH-8542 Wiesendagen (CH)
(74) Vertreter: Misrachi, Alfred

(56) Entgegenhaltungen:
- US-A- 4 655 771
- US-A- 5 071 407

## Beschreibung

Die Erfindung betrifft einen Katheter mit einer selbst expandierenden zylindrischen Gefässstütze aus einem durchlässigen Netz sich überkreuzender steifer Fäden, wobei die Gefässstütze bei ihrem Einsatz sich aus einem gespannten Zustand mit keinem Umfang durch ihre radiale Elastizität aus eigener Kraft aufweitet in einen die Gefässwand stützenden entspannten Zustand mit über seine Länge gleichbleibendem grossem Umfang, mit einem verschieblichen schlauchförmigen äusseren Katheterschaft, der an seinem distalen Ende die gespannte Gefässstütze in sich aufnimmt und aus dem die Gefässstütze zu ihrem Einsatz freigegeben werden kann, mit einem verschieblichen Innenkatheter im Innern des schlauchförmigen äusseren Katheterschafts, der die Gefässstütze an ihrem proximalen Ende axial abstützt, wobei zur Freigabe der Gefässstütze der äussere Katheterschaft gegenüber dem Innenkatheter zurückgezogen wird, wobei die Gefässstütze (5) an ihrem proximalen Ende eingespannt so gesichert ist, dass sie einen mit der Gefässstütze (5) sich selbst öffnenden durchlässigen Netzkegel (7) bildet, dessen Radius langsam zum Radius der entspannten Gefässstütze (5) ansteigt, wobei die Gefässstütze (5) mit Hilfe des Netzkegels (7) fest am Innenkatheter (4) verankert ist und wobei am proximalen Ende des Netzkegels (7) die Gefässstütze (5) auf den Aussendurchmesser des Innenkatheters (4) zusammengezogen ist.

Katheter mit einer selbst expandierenden zylindrischen Gefässstütze sind beispielsweise aus der US 4 655 771 bekannt. Sie werden eingesetzt, um Gefässstützen, Gefässendoprothesen, sogenannte Stents, in Gefässen des menschlichen Körpers einzupflanzen. Ein besonderer Einsatzbereich für diese Gefässstützen hat sich in letzter Zeit in Zusammenhang mit dem verstärkten Einsatz der perkutanen transluminalen Koronar-Rekanalisation (PTCA) ergeben. Bei dieser Technik wird ein Katheter durch einen Einstich in die Haut in ein Blutgefäss eingeführt und über das Blutgefäss bis zu einer beispielsweise arteriosklerotischen Verengung in den Herzkranzgefässen vorgeschoben. Am Ende des Katheters ist ein auffüllbarer Ballon angebracht. Dieser Ballon wird unter Druck gesetzt und die Verengung in dem Blutgefäss wird durch diesen Druck aufgeweitet. Danach kann der Ballon wieder entleert werden und der Katheter aus dem Körper entfernt werden. In der Mehrzahl der Fälle bleibt das Blutgefäss dann weiterhin für den Blutdurchfluss offen.

Eine Hauptkomplikation bei dieser Technik besteht jedoch darin, dass durch diese Massnahme Teile der Intima, der innersten Schicht der Gefässwand, sich von der Gefässwand loslösen und dann mehr oder weniger stark den Durchfluss im Gefäss behindern. Im schlimmsten Fall kann die losgelöste Gefässwand wie eine Ventilklappe wirken, die den Durchfluss ganz versperrt. An bestimmten Behandlungsorten, z.B. in den Koronararterien, führt das zu einer kritischen Situation, die eine notfallmässige Bypass-Operation mit hohem Risiko für den Patienten notwendig macht. Aber auch an anderen Behandlungsorten und bei weniger ungünstigem Verlauf der Komplikation ist auf jeden Fall durch diese Komplikation der angestrebte Erfolg der Behandlung verhindert.

Im Falle solcher Komplikationen werden nun seit einiger Zeit die beispielsweise aus der US 4 655 771 bekannten Gefässstützen an der behandelten Stelle in das Gefäss eingesetzt um das Gefäss von innen offen zu halten. Dazu wird durch denselben Einstich, der schon für den Ballonkatheter benutzt worden ist, ein Katheter in das Blutgefäss eingeführt. In diesem Katheter liegt innen an seinem vom Bediener entfernten, distalen Ende die Gefässstütze. Die Gefässstütze ist zylindrisch und besteht aus einem Netz sich überkreuzender steifer Fäden. Sie ist vom selbstexpandierenden Typ, d.h. sie ist in gespanntem Zustand in den Katheter eingelegt und entspannt sich ohne Hilfe aus eigener Kraft. Andere Gefässstützentypen müssen z.B. durch einen innenliegenden Ballon in ihren aufgeweiteten Zustand gebracht werden. Am Behandlungsort wird die Gefässstütze durch Zurückziehen des Aussenkatheters aus dem Katheter freigegeben und vom Katheter gelöst. Ein im Katheter liegender verschieblicher Innenkatheter dient der Gefässstütze als Widerlager solange der Aussenkatheter zurückgezogen wird. Die Gefässstütze verbleibt nach ihrer Freigabe im Gefäss, sie stützt das Gefäss permanent. Der Katheter dagegen wird wie üblich zurückgezogen und die Einstichstelle in das Gefäss wird verschlossen.

Solche Gefässstützen erfüllen insofern ihren Zweck, als sie die losgelöste innerste Gefässschicht, die Intima, wieder an die Gefässwand drücken und damit das Gefäss für den Durchfluss offen halten. Schwierigkeiten treten aber insofern auf, als diese Gefässstützen Blutgerinnsel verursachen können. Dieser Gefahr muss mit hohen, potentiell gefährlichen Dosen von gerinnungshemmenden Medikamenten begegnet werden. Nach einigen Wochen wird die Gefässstütze dann von der Gefäss-Innenhaut, dem Endothel überwachsen und die Gefahr von Blutgerinnseln ist dadurch weitgehend gebannt. Jetzt tritt eine neue Schwierigkeit auf. Es zeigt sich nämlich, dass die Gewebezellen, die durch das Einbringen der Gefässstütze in ihrem Wachstum angeregt wurden, in einigen Fällen nicht mehr aufhören zu wachsen. Es kann daher zu einem erneuten teilweisen oder vollständigen Verschluss des Gefässes kommen.

Gleichzeitig ist es bekannt, dass eine losgelöste Intima schon in relativ kurzer Zeit wieder mit der Gefässwand verbunden und ausgeheilt werden kann. Teilweise reicht dazu ein erneutes kurzzeitiges Auffüllen des Ballons am Ende des beschriebenen Ballonkatheters. Während der Ballon gespannt ist, ist aber in dem entsprechenden Gefäss der Blutfluss unterbrochen, so dass diese Methode nicht an allen Behandlungsorten einsetzbar ist. Ausserdem werden die Ausheilzeiten durch eventuell während der Behandlung notwendige gerinnungshemmende Medikamente verlängert.

Um den geschilderten Schwierigkeiten aus dem Wege zu gehen, sind deshalb schon Katheter vorgeschlagen worden, bei denen die Gefässstütze nur vorübergehend die Gefässwand abstützt und danach wieder aus dem Gefäss entfernt werden kann.

Ein Beispiel dafür ist in der EP 0 321 912 A1 dargestellt. Es handelt sich dabei um eine Gefässstütze aus einem Netzschlauch aus verwobenen Drähten, der in die Länge gestreckt in das Gefäss eingeführt wird. Am Behandlungsort werden die beiden Enden des Netzschlauches gegeneinander bewegt, und das Netz zwischen den Enden beult sich aus zu einer Hohlform, die sich an die Gefässinnenwand legt und sie stützt. Das Netz, aus dem die Gefässstütze besteht, ist also in diesem Fall nicht selbstexpandierend, sondern es hat seine entspannte Lage im gestreckten Zustand. In diesem entspannten gestreckten Zustand mit kleinem Umfang wird die Gefässstütze in das Gefäss eingeführt und nach dem Gebrauch auch wieder aus dem Gefäss entfernt. Je nachdem wie stark die Enden des Netzschlauches aufeinander zu bewegt werden, entsprechend stark ist der Druck der Hohlform auf die Gefässwand. Nachteilig an dieser Ausführung ist allerdings, dass die einzelnen Drähte knicken können, wenn die Enden des Netzschlauches zu stark gegeneinander gezogen werden und die Drähte im Gefäss nicht ausweichen können. Mit geknickten Drähten ist der Katheter nur mit Komplikationen aus dem Gefäss zu entfernen, weil die Betätigungselemente des Netzes nur bedingt Druckkräfte übertragen können um das Netz wieder in die gestreckte Form mit kleinem Umfang zurück zu bringen. Nachteilig ist ausserdem, dass das Netz an seinen beiden Enden zusammengebündelt ist, das Netzgewebe muss also beim Einsatz im Blutgefäss vom durchfliessenden Blut zweimal passiert werden, am vorderen Ende der Gefässstütze und am hinteren Ende der Gefässstütze. Nachteilig ist ausserdem, dass die Betätigungskräfte zum Offenhalten des Netzschlauches während der Behandlungsdauer über eine relativ weite Strecke von aussen aufrecht erhalten werden müssen. Es können sich dabei Übertragungsfehler einstellen wenn zum Beispiel der Katheter zwischen dem Einstich in die Haut und dem Behandlungsort bewegt wird.

Ein weiteres Beispiel für eine wieder aus dem Körper entfernbare Gefässstütze ist in der WO 91/07 928 gezeigt. In diesem Fall wird die Gefässtütze aus einem schraubenförmig gewundenen einzigen Draht gebildet. Der Draht ist gestreckt in einem dünnen Katheterrohr aufgenommen und wird daraus hervorgeschoben. Sobald der Draht vorne aus dem dünnen Katheterrohr austritt, nimmt er wegen der in ihm vorgesehenen Spannungen eine Spiral- oder Schraubenform an. Die einzelnen Windungen der Schraubenform drängen radial nach aussen und stützen die Gefässwand. Zum Entfernen der Gefässstütze wird der Draht wieder in den Katheter zurückgezogen. Der Draht nimmt dabei wieder seine gestreckte Form ein. Diese Gefässstütze ist damit vom selbstexpandierenden Typ. Beim selbstexpandierenden Typ droht nicht die Gefahr, dass übermässige Bedienungskräfte die Gefässstütze beeinträchtigen oder unbrauchbar machen. Die Durchflussbedingungen für das Blut sind bei dieser Gefässstütze denkbar günstig, weil die Schraubenform am katheterfernen Ende offen ist und damit die Windungen der Schraubenform nur einmal durchflossen werden müssen. Die Verwendung nur eines Drahtes bringt allerdings den Nachteil mit sich, dass die Drähte der Gefässstütze sehr eng nebeneinander angeordnet werden müssen um die Gefässwand flächenmässig und damit wirkungsvoll abzustützen. In einer Schraubenform mit nur einem Draht haben aber die Windungen untereinander keinen Zusammenhalt, der einen gleichmässig engen Abstand der Windungen sicherstellen würde. Es können somit Lücken in der Abstützung der Gefässwand entstehen. Ein unangenehmer Nachteil liegt auch darin, dass die schraubenförmige Gefässstütze beim Einsetzen und beim Entfernen aus dem Gefäss nicht stationär bleibt. Beim Einsetzen und beim Entfernen der Gefässstütze muss der jeweils entspannte Teil der Schraubenfeder sich gegenüber dem Draht im Katheter drehen um den unterschiedlichen Zustand des Drahtes auszugleichen. Beim Setzen der Gefässstütze kann dabei das freie sich drehende Ende des Drahtes in der Gefässwand Verletzungen hervorrufen. Vor allem aber kann beim Setzen die schraubenförmige Gefässstütze durch ihre Drehung unter einen losgelösten Lappen der Gefässwand wandern und damit die Erfüllung ihrer Aufgabe verhindern. Diese Gefässstütze ist daher nicht sicher wirksam wie z.B. die bekannte permanent im Gefäss verbleibende Gefässstütze. Schwierigkeiten bereiten ausserdem die hohen Reibungskräfte des gespannten Drahtes in dem dünnen Katheter. Zum Ausstossen der Gefässstütze aus dem Katheter müssen daher grosse Widerstände überwunden werden, die durch das Anpressen des Drahtes an die Katheterwand während des Ausstossens sogar noch anwachsen.

Ein weiteres Beispiel für eine wieder aus dem Gefäss entfernbare Gefässstütze ist in der EP 0 423 916 A1 veröffentlicht. Es handelt sich um ein in Form eines Schlauchmantelstücks zusammengesetztes Scherengitter aus einem nichtrostenden Stahldraht. Diese Gefässstütze ist ebenfalls vom selbstexpandierenden Typ und wird genau wie die Gefässstütze nach der US 4 655 771 durch Zurückziehen eines Aussenkatheters gegenüber einem Innenkatheter in das Gefäss eingesetzt. Am bedienungsnahen, proximalen Rand des zu einem Schlauchmantelstück zusammengesetzten Scherengitters ist am Rand des Schlauchmantel ein Faden angebracht. Mit diesem Faden kann der Schlauchmantel an seinem proximalen Ende zusammengeschnürt werden. Um dies zu erreichen, werden beide Enden dieses Fadens bis ausserhalb des Körpers geführt und dort lose gesichert solange die Gefässstütze im Gefäss ist. Soll die Gefässstütze entfernt werden, wird über diese beiden Fäden ein neuer Katheter bis zur Gefässstütze vorgeschoben und durch Zug an den Fäden wird das proximale Ende der Stütze zusammengeschnürt. Sodann wird ein zweiter entsprechend grösserer Katheter über den ersten geschoben. Die Gefässstütze wird jetzt mit den Fäden so weit zusammengezogen bis sie in den grösseren Katheter passt und in ihn hineingezogen werden kann. Danach werden beide Katheter zusammen mit der Gefässstütze entfernt. Vorteilhaft an dieser Anordnung ist der gute Durchfluss der sich bei eingesetzter Gefässstütze ergibt, weil das Schlauchmantelstück an beiden Enden offen ist. Nachteilig ist der grosse Aufwand der für diese Anordnung getrieben werden muss. Die Bedienung ist sehr umständlich durch die Handhabung der Fäden und das Setzen mindestens eines neuen Katheters, der z.B. auch über die Fäden geschoben werden muss. Der instrumentelle Aufwand ist ebenfalls sehr hoch, es muss mindestens ein zusätzlicher Katheter vorgesehen werden, der die Gefässstütze wieder aufnimmt. Der zum Setzen der Gefässstütze benutzte Katheter ist dazu zu klein. Beim Einsatz der Gefässstütze in den Koronararterien ist beim Einziehen der Gefässstütze in den grösseren Katheter ausserdem mit Schwierigkeiten zu rechnen, weil die Koronararterien ständig in Bewegung sind. Die Schwierigkeiten kommen dann daher, dass die am proximalen Ende zusammengezogene Gefässstütze nicht immer genau mittig zum grösseren Katheter liegt und die Gefässstütze sich auch nicht selbst gegenüber dem grösseren Katheter zentriert. Die Gefässstütze bleibt am Rand des grösseren Katheters hängen.

Aus der US 5 071 407 ist auch ein Katheter mit einem selbstexpandieren Drahtgewebe bekannt, bei dem das Drahtgewebe dazu dient, den Katheter im Gewebe zu verankern. Das selbstexpandierende Drahtgewebe ist an seinem proximalen Ende zusammengefasst und mit einem Fixationsring am distalen Ende eines Innenkatheters eingespannt. Vor dem Gebrauch dieser Vorrichtung liegt das selbstexpandierende Drahtgewebe im komprimierten Zustand in einem Aussenkatheter. Zur Verankerung des Katheters in einem Gefäss wird der Aussenkatheter zurückgezogen und das Drahtgewebe expandiert dadurch bis zur Anlage an die Gefässwand. Wird der Aussenkatheter wieder nach distal geschoben, kann das Drahtgewebe wieder zurückgefaltet werden und in den Aussenkatheter hineingezogen werden, sodass der gesamte Katheter wieder aus dem Gefäss entfernbar ist. Der Fixationsring zur Einspannung des Drahtgewebes auf dem Innenkatheter bildet möglicherweise ein Hindernis beim Vorschieben des Aussenkatheters, ausserdem führt er zu einer Durchmesservergrösserung des gesamten Instruments und mit seiner Querschnittsfläche behindert er den Fluid-Durchfluss im Gefäss. Wegen der Vorspannung des selbstexpandierenden Drahtgewebes ist ein solcher Fixationsring auch schwierig mit der notwendigen Zuverlässigkeit zu montieren und es ist aufwending bei einem solchen Ring einen auf dem ganzen Umfang gleichförmigen Anpressdruck zu erreichen damit alle Drähte gleich gut festgehalten werden.

Die Erfindung hat sich daher die Aufgabe gestellt, einen Katheter mit einer in sich zusammengehaltenen und beim Freigeben sich nicht verdrehenden Gefässstütze so weiter zu entwickeln, dass die Gefässstütze nach einer vorübergehenden Einsatzzeit zuverlässig ohne Schwierigkeiten wieder aus dem Gefäss entfernbar ist, wobei die weiterzuentwickelnde Gefässstütze vom selbstexpandierenden Typ sein soll um für die entfernbare Gefässstütze eine Bedienung ohne Risiko zu gewährleisten, wobei der Katheter möglichst einfach aufgebaut und einfach und leicht zu bedienen sein soll und wobei während der vorübergehenden Einsatzzeit der Gefässstütze im Gefäss der Strom des Mediums möglichst wenig behindert werden soll. Gleichzeitig soll ein Verfahren zur Herstellung dieses Katheters angegeben werden.

Diese Aufgabe wird dadurch gelöst, dass die Gefässstütze (5) am proximalen Ende des Netzkegels durch eine Schicht Bindemittel mit dem Innenkatheter verbunden wird.

Durch diese Massnahmen ist ein Katheter mit Gefässstütze herstellbar, der nach seinem Einsatz zusammen mit der Gefässstütze zuverlässig und ohne Schwierigkeiten wieder aus dem Gefäss entfernt werden kann. Dazu muss nur der äussere Katheterschaft gegenüber dem verschieblichen Innenkatheter vorgeschoben werden und gegebenenfalls, wenn die Gefässstütze sich von der Gefässwand gelöst hat, der verschiebliche Innenkatheter zurückgezogen werden. Der äussere Katheterschaft gleitet dann von der Spitze her kommend aussen auf dem am Innenkatheter verankerten Netzkegel entlang und zwingt dadurch den von der Gefässstütze gebildeten Netzkegel und die Gefässstütze wieder in ihre gespannte Form zurück. Durch die Kegelform am proximalen Ende der Gefässstütze kann die Gefässstütze bei diesem Vorgang sich nicht verhaken, die Gefässstütze zentriert sich selbst gegenüber dem äusseren Katheterschaft. Dadurch kann der äussere Katheterschaft von Anfang an glatt über die Gefässstütze hinweg geschoben werden, bzw. die Gefässstütze kann glatt in den Katheter zurückgezogen werden. Die zusammenziehenden Kräfte sind dabei durch die Form des Netzkegels gross genug aber auch so gleichmässig verteilt, dass die Gefässstütze wieder auf den ursprünglich eingenommenen kleinen Umfang zurückgebracht werden kann und wieder in denselben ursprünglich benutzten äusseren Katheterschaft passt. Wenn die Gefässstütze wieder mit ihrer ganzen Länge innerhalb des äusseren Katheterschaftes liegt, kann der Katheter dann insgesamt mit der Gefässstütze abgezogen werden. Es entsteht also durch diese Massnahmen nicht nur ein denkbar einfach aufgebauter Katheter ohne zusätzliche neue Bauteile, sondern auch ein einfach, sicher und leicht zu bedienender Katheter. Es treten im wesentlichen nur die Reibungskräfte auf, die von der herkömmlichen permanent zu setzenden Gefässstütze bekannt sind und die demgegenüber geringen Kräfte zum Zusammenziehen der Gefässstütze. Die Elemente des Bedienungsablaufes sind denkbar einfach und sind ebenfalls von der herkömmlichen permanent zu setzenden Gefässstütze bekannt. Gleichzeitig wird durch die Verwendung des durchlässigen Netzkegels als Befestigungsmittel der Gefässstütze am Innenkatheter sichergestellt, dass der Strom des Mediums im Gefäss möglichst wenig behindert wird.

Wenn am proximalen Ende des Netzkegels die Gefässstütze auf den Aussendurchmesser des Innenkatheters zusammengezogen ist und durch eine Schicht Bindemittel mit dem Innenkatheter verbunden ist, ergibt sich ein besonders einfach aufgebauter und einfach herzustellender Katheter mit einem glatten Übergang vom Innenkatheter auf das proximale Ende des Netzkegels.

Eine weitere besondere Ausführungsform ergibt sich, wenn der Innenkatheter schlauchförmig ausgebildet ist und innerhalb der Gefässstütze endet. Der schlauchförmige Innenkatheter kann beispielsweise einen Führungsdraht aufnehmen, damit die Gefässstütze entlang eines im Körper schon gelegten Führungsdrahtes vorgeschoben werden kann. Wenn ausserdem der Innenkatheter distal nicht aus der Gefässstütze herausragt, sondern innerhalb der Gefässstütze endet, kann der Katheter ausser zum Einsatz einer wieder entfernbaren Gefässstütze gleichzeitig auch noch zu einer in der DE GM 89 10 603.2 geschilderten Methode zur Entfernung von Blutgerinnseln aus Arterien und Venen mit Hilfe eines sog. Fogarty-Katheters eingesetzt werden. In seinem inneren Lumen nimmt der Innenkatheter dazu in diesem Fall nicht einen Führungsdraht, sondern einen Ballonkatheter auf, den Fogarty-Katheter. Dieser Ballonkatheter kann dann gegebenenfalls seinerseits mit einem inneren Führungsdraht ausgerüstet sein. Der Ballon dieses Fogarty-Katheters ist im Gegensatz zu den Ballonen der vorher erwähnten Ballonkatheter elastisch, d.h. er kann sich weitgehend dem Gefässdurchmesser anpassen. Der Fogarty-Katheter wird aus dem Innenkatheter heraus durch das Blutgefäss vorgeschoben und durch ein in dem Blutgefäss befindliches Blutgerinnsel hindurchgestossen. Hinter dem Blutgerinnsel wird der Ballon des Fogarty-Katheters aufgeblasen und der Fogarty-Katheter wird zurückgezogen. Der aufgeblasene Ballon schiebt dabei das Blutgerinnsel im Blutgefäss vor sich her, bis das Blutgerinnsel zwischen dem Ballon und dem erfindungsgemässen Katheter gefangen ist. Um die Methode nach der DE GM 89 10 603.2 auszuführen, muss jetzt das distale Ende der Gefässstütze als Fangkorb benutzt werden. Die Gefässstütze wird dazu vorgeschoben, bis ihr distales Ende aus dem äusseren Katheterschaft herausgetreten ist und sich auf den Durchmesser des Gefässes aufgeweitet hat. Dadurch bildet sich am distalen Ende des äusseren Katheterschafts ein Fangkorb, in den das Blutgerinnsel mit Hilfe des Fogarty-Ballons hineingeschoben werden kann. Der Fangkorb, beziehungsweise die als Fangkorb benutzte Gefässstütze wird dann in den äusseren Katheterschaft des erfindungsgemässen Katheters wieder eingezogen. Dabei zieht sich der Fangkorb wieder zusammen, er presst das Blutgerinnsel aus und trennt flüssige Bestandteile von faserigen Bestandteilen. Beim weiteren Zurückziehen des Innenkatheters mit dem Fangkorb wird die als Fangkorb dienende Gefässstütze zusammen mit den faserigen Bestandteilen des Blutgerinnsels durch den äusseren Katheterschaft wieder aufgenommen.

Zusammen mit dem Katheter kann jetzt das Blutgerinnsel entfernt werden. Für die Methode nach der DE GM 89 10 603.2 galt es an sich als vorteilhaft, den Fangkorb mit einer undurchlässigen Beschichtung oder Membran zu überziehen, damit das Blutgerinnsel nicht aus dem Fangkorb entweicht. Es hat sich aber gezeigt, dass die zum Katheterschaft hin kleiner werdenden Maschen bei der Gefässstütze des erfindungsgemässen Katheters ausreichen, um die faserigen Bestandteile des Blutgerinnsels zurückzuhalten und dass der erfindungsgemässe Katheter daher auch für die Methode nach der DE GM 89 10 603.2 eingesetzt werden kann. Mit der angegebenen Massnahme entsteht also ein Mehrzweckinstrument, das einerseits als wieder entfernbare Gefässstütze gebraucht werden kann, andererseits aber auch als Gerät zum Entfernen von Blutgerinnseln aus Arterien und Venen.

Der äussere Katheterschaft bei dem angegebenen Mehrzweckinstrument kann auch mit einer Schutzkappe versehen sein, die von der Gefässstütze durchstossen werden kann. Der Katheter ist dann vorne nicht mehr ganz offen wenn er durch das Gefäss geschoben wird. Vor allem werden Verletzungen der Gefässwand durch den vorderen Rand des äusseren Katheterschafts vermieden. Trotz dieses Schutzes beim Vorschieben lässt die Gefässstütze am Behandlungsort sich freisetzen, da die Schutzkappe von der Gefässstütze durchstossen werden kann.

Ein besonders vorteilhaftes Verfahren zur Herstellung eines erfindungsgemässen Katheters ergibt sich, wenn die Spitze des Netzkegels der Gefässstütze durch einen Schrumpfschlauch auf dem Aussendurchmesser des Innenkatheters zusammengehalten wird während der Netzkegel mit dem Innenkatheter verbunden wird. Dadurch wird durch eine einfache Massnahme das Netz der Gefässstütze von allen Seiten gleichmässig eingespannt und die Verbindung durch Wärmebehandlung oder mit Hilfe von Bindemitteln kann ungestört hergestellt werden. Ein besonderer Vorteil in dieser Methode liegt darin, dass die Einspannung eine konstante Kraft ausübt, auch wenn der eingespannte Gegenstand nachgibt oder gar im Durchmesser kleiner wird. Diese Eigenart des vorgeschlagenen Verfahrens ist wichtig, wenn die Verbindung durch Wärmebehandlung hergestellt wird, aber auch wenn die Verbindung mit Bindemitteln der kleinstmögliche Durchmesser der fertigen Verbindung erzielt werden soll.

Nachfolgend wird die Erfindung anhand eines Ausführungsbeispiels in den Zeichnungen näher erläutert. Es zeigen:
- Fig. 1: eine Ansicht eines Katheters nach der Erfindung mit freigesetzter Gefässstütze in einem schematisch angegebenen Gefäss
- Fig. 2: einen Querschnitt durch einen Katheter nach der Erfindung mit teilweise eingespannter und teilweise freigegebener Gefässstütze
- Fig. 3: eine Ansicht eines Katheters nach der Erfindung mit teilweise freigegebener Gefässstütze.

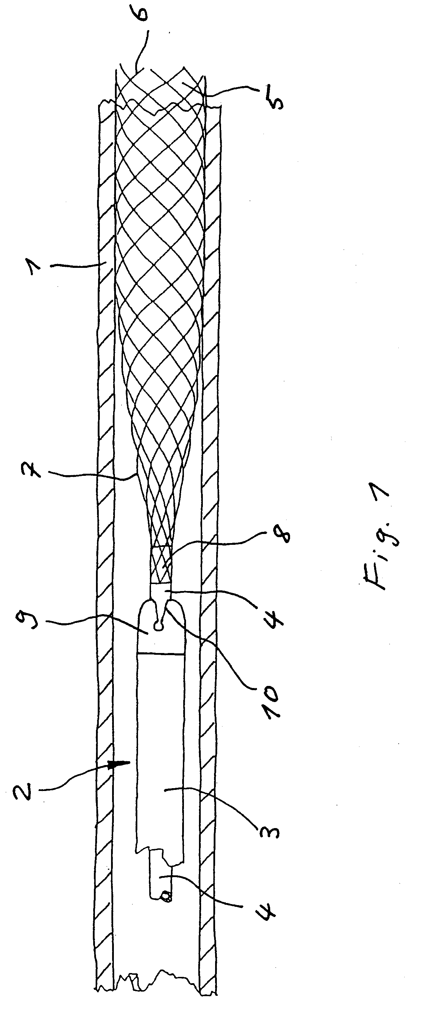

In Fig. 1 ist mit 1 idealisiert ein Gefäss z.B. eines menschlichen Körpers dargestellt. Das Gefäss kann Flüssigkeiten, z.B. Blut aufnehmen, es kann aber auch Luft führen, sodass das Gefäss auch eine Luftröhre darstellen kann. In diesem Gefäss liegend ist das bedienungsferne, distale Ende eines Katheters 2 dargestellt. Der Katheter 2 ist an geeigneter Stelle, z.B. an einer Punktion, in das Gefäss eingeführt worden und ist von ausserhalb des Körpers bis zu der dargestellten Stelle im Gefäss vorgeschoben worden. Der Katheter 2 besteht aus einem schlauchförmigen äusseren Katheterschaft 3 und einem in diesem liegendenden verschieblichen Innenkatheter 4. In der in Fig. 1 dargestellten Lage ist der Innenkatheter 4 aus dem distalen Ende des äusseren Katheterschaftes 3 ein Stück herausgeschoben.

Ausser dem Katheter 2 liegt in dem Gefäss 1 auch noch eine Gefässstütze 5. Die Gefässstütze 5 ist aus einem durchlässigen Netz sich überkreuzender steifer Fäden 6 hergestellt. Die Steifigkeit der Fäden 6 ist so ausgewählt, dass die Gefässstütze 5 bei ihrem Einsatz sich aus einem gespannten Zustand mit kleinem Umfang durch ihre radiale Elastizität aus eigener Kraft aufweitet in einen die Gefässwand stützenden entspannten Zustand mit über die Länge gleichbleibendem grossem Umfang.

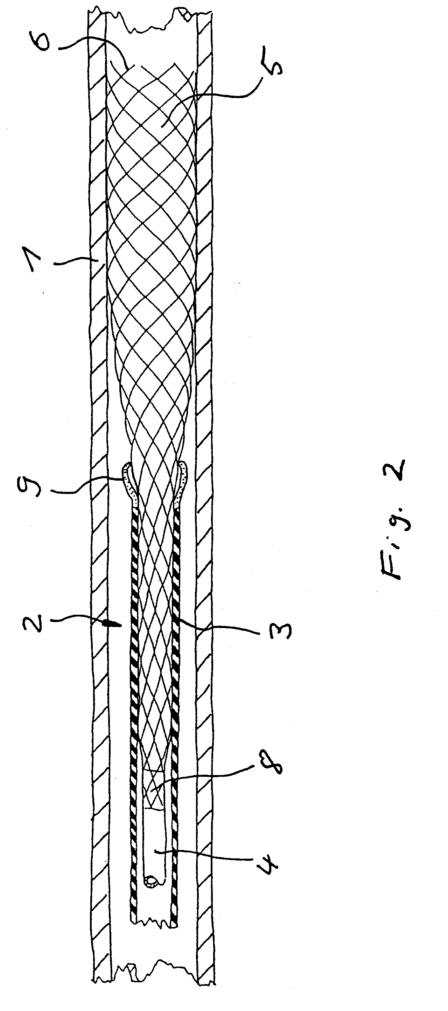

In Fig. 2 ist ein Teil der Gefässstütze 5 in gespanntem Zustand zu sehen. Der gespannte Teil der Gefässstütze 5 liegt in der linken Figurenhälfte auf kleinen Umfang zusammengezogen am distalen Ende im schlauchförmigen äusseren Katheterschaft 3 gefangen. Um die Gefässstütze 5 zu ihrem Einsatz in ihren entspannten Zustand freizugeben in dem sie selbstexpandierend sich aufweitet, sich an die Gefässwand anlegt und diese stützt, muss der äussere Katheterschaft 3 gegenüber dem Innenkatheter 4 zurückgezogen werden. Der Innenkatheter 4 muss dabei die im äusseren Katheterschaft 3 an der Innenwand anliegende Gefässstütze 5 an ihrem proximalen Ende axial abstützen, damit eine Relativbewegung zwischen äusserem Katheterschaft 3 und Gefässstütze 5 zustande kommen kann.

An ihrem distalen Ende ist die Gefässstütze 5 offen, sodass das Medium das Gefäss 1 frei durchströmen kann. Besonders in Fig. 1 ist zu sehen, dass die Gefässstütze jedoch an ihrem proximalen, bedienungsnahen Ende eingespannt so gesichert ist, dass sie einen durchlässigen Netzkegel 7 bildet. Der Netzkegel 7 ist aus denselben steifen Fäden 6 hergestellt wie die Gefässstütze 5, sodass er sich zusammen mit der Gefässstütze selbst öffnet. Der Radius dieses Netzkegels 7 steigt langsam zum Radius der Gefässstütze 5 an. An seinem proximalen Ende ist der Netzkegel 7 auf den Aussendurchmesser des Innenkatheters 4 zusammengezogen. Er ist dort durch eine Schicht Bindemittel mit dem Innenkatheter verbunden. Die Gefässstütze 5 ist daher an der Verbindungsstelle 8 mit Hilfe des Netzkegels 7 fest am Innenkatheter 4 verankert.

Ein vorteilhaftes Verfahren, einen Katheter 2 nach der Erfindung herzustellen besteht darin, die Gefässstütze 5 an ihrem proximalen Ende mit einem Schrumpfschlauch auf dem Aussendurchmesser des Innenkatheters 4 zusammenzuhalten, während der Netzkegel 7 mit dem Innenkatheter 4 verbunden wird. Der Schrumpfschlauch wird in seinem Rohzustand in dem er noch einen grossen Durchmesser hat, über die Gefässstütze 5 gezogen. Der Schrumpfschlauch wird dann erwärmt, er zieht sich dadurch zusammen und schnürt damit auch die Gefässstütze 5 ein, die er umgibt. Abmessungen und Material des Schrumpfschlauches sowie die Wärmezufuhr zum Schrumpfschlauch können so ausgewählt werden, dass der Schrumpfschlauch die Gefässstütze 5 auf den Aussendurchmesser des Innenkatheters 4 zusammenzieht und dort zusammenhält. In diesem Zustand kann dann die Gefässstütze 5 mit dem Innenkatheter 4 verschweisst werden oder eine vorher angebrachte oder durch Kapillarwirkung unter den Schrumpfschlauch in die Verbindungsstelle 8 hinein wandernde Schicht Bindemittel kann aushärten. Nach diesem Vorgang kann der Schrumpfschlauch wieder entfernt werden, sodass, wie in den Fig. 1 und 2 dargestellt, eine glatte Verbindungsstelle 8 übrig bleibt, die, je nach gewähltem Verfahren, im wesentlichen den Durchmesser des Innenkatheters 4 aufweisen kann.

Zum Gebrauch liegt die Gefässstütze 5 zunächst gespannt am distalen Ende innerhalb des äusseren Katheterschaftes 3 im Katheter 2. Der Innenkatheter 4 ist zurückgezogen gegenüber dem äusseren Katheterschaft 3 und die Gefässstütze 5 legt sich gegen die Innenwand des äusseren Katheterschaftes 3 wie im linken Teil der Fig. 2. Der Innenkatheter ist soweit zurückgezogen, dass auch das distale Ende der Gefässstütze 5 innerhalb des äusseren Katheterschaftes 3 liegt. In diesem Zustand wird der Katheter 2 in das Gefäss 1 eingeführt und in diesem Gefäss 1 vorgeschoben. Wenn das distale Ende des Katheters 2 die Behandlungsstelle passiert hat, wird der äussere Katheterschaft 2 gegenüber dem stationär gehaltenen Innenkatheter 4 zurückgezogen. Durch die Verbindung der Gefässstütze 5 mit dem stationär gehaltenen Innenkatheter 4 kommt eine Relativbewegung zwischen Gefässstütze und äusserem Katheterschaft 3 zustande. Dadurch wird die Gefässstütze 5 langsam Stück für Stück, von ihrem distalen Ende her anfangend, freigegeben. Sie tritt aus dem distalen Ende des äusseren Katheterschaftes 3 aus und weitet sich langsam auf in ihren entspannten Zustand, in dem sie sich an die Gefässwand anlegt und sie stützt. Fig. 2 zeigt den Zustand, wenn der äussere Katheterschaft 3 ungefähr die halbe Länge zurückgezogen ist. Die Gefässstütze wird nun so weit wie erforderlich freigegeben, z.B. bis die Verbindungsstelle 8 aus dem distalen Ende des äusseren Katheterschaftes 3 ausgetreten ist wie in Fig. 1.

Soll nun die Gefässstütze 5 wieder aus dem Gefäss 1 entfernt werden, so muss der äussere Katheterschaft 3 gegenüber dem Innenkatheter 4 lediglich wieder vorgeschoben werden. Durch die Verbindung der Gefässstütze 5 mit dem Innenkatheter 4 ergibt sich auch daraus wieder eine Relativbewegung zwischen Gefässstütze und äusserem Katheterschaft 3. Durch die Verbindung der Gefässstütze 5 mit dem Innenkatheter 4 mit Hilfe eines Netzkegels 7, dessen proximales Ende am Innenkatheter 4 befestigt ist, gleitet der Katheterschaft 3 aussen auf dem Netz der Gefässstütze 5 entlang und zwingt dadurch den vom Innenkatheter 4 aus ansteigenden Netzkegel 7 und die daran anschliessende Gefässstütze 5 wieder in ihre gespannte Form zurück in der sie wieder vom Katheterschaft 3 aufgenommen werden kann. Sobald die Gefässstütze sich von der Gefässwand gelöst hat, kann auch der Innenkatheter 4 gegenüber dem äusseren Katheterschaft 3 zurückgezogen werden. Die Gefässstütze 5 kann auf diese Weise wieder ganz in den äusseren Katheterschaft 5 zurückgezogen werden und der Katheter 2 kann aus dem Gefäss 1 entfernt werden.

Im gezeigten Ausführungsbeispiel ist der Innenkatheter 4 ebenfalls schlauchförmig ausgebildet und der Innenkatheter 4 endet distal hinter der Verbindungsstelle 8. Im Gebrauch nimmt der schlauchförmige Innenkatheter 4 beispielsweise einen Führungsdraht auf. Dies kann ein Führungsdraht sein, der zum Einsatz des Katheters 2 im Körper vorgeschoben wird und dem der Katheter 2 dann folgen kann. Es kann aber auch ein Führungsdraht sein, der von einer vorhergehenden Behandlung schon im Körper liegt, beispielsweise von der Behandlung mit einem Ballonkatheter.

In Fig. 1 und 2 ist sichtbar, dass der Innenkatheter 4 direkt hinter der Verbindungsstelle 8 endet. Der Katheter 2 kann in diesem Fall ausser zum Einsatz einer wieder entfernbaren Gefässstütze 5 auch zu einem in der Einleitung näher dargestellten Verfahren zur Entfernung von Blutgerinnseln aus Arterien und Venen nach der DE GM 89 10 603.2 eingesetzt werden. Das Verfahren ist in der Einleitung und in der angegebenen Fundstelle eingehend beschrieben, auf eine Wiederholung der Beschreibung an dieser Stelle wird verzichtet. Um den Katheter 2 als Mehrzweckinstrument einsetzen zu können, muss der Innenkatheter 4 nicht direkt hinter der Verbindungsstelle 8 enden, er kann auch im Abstand vom distalen Ende der Gefässstütze 5 enden, es reicht, wenn er auf jeden Fall innerhalb der Gefässstütze 5 endet.

Am distalen Ende des äusseren Katheterschafts 3 ist eine Schutzkappe 9 am Katheterschaft 3 angebracht. Sie ist thermisch oder mit Bindemitteln mit dem Katheterschaft 3 verbunden, sie kann aber auch einstückig aus dem Material des Katheterschaftes 3 selbst geformt sein. Die Schutzkappe 9 ist elastisch oder bleibend verformbar. Bei der Einführung des Katheters 2 in das Gefäss 1 ist diese Schutzkappe 9 geschlossen. Sie verhindert damit allzustarkes Eindringen von Flüssigkeit aus dem Gefäss in den Katheter 2. Die Kappe 9 besitzt eine gerundete Spitze. Durch die Abdeckung des distalen Randes des äusseren Katheterschaftes 3, die Rundung an der Spitze und durch ihre Nachgiebigkeit verhindert die Kappe 9 Verletzungen der Gefässwand beim Vorschieben des Katheters 2.

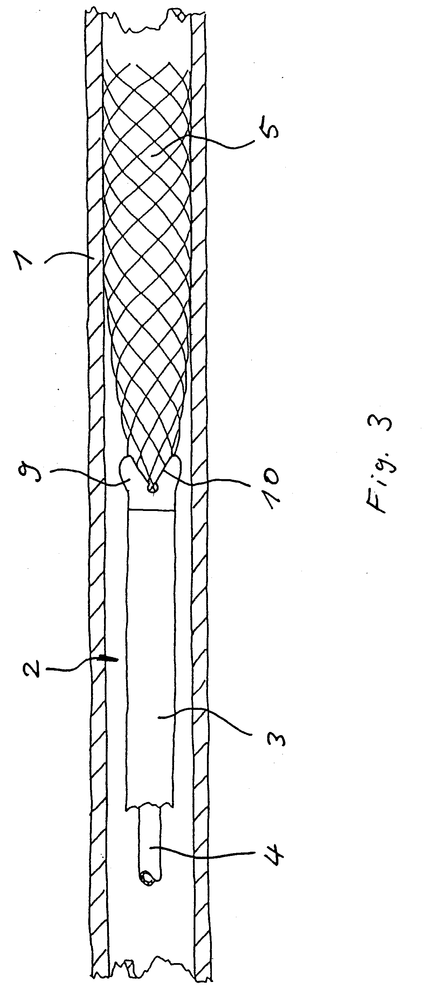

Wenn der Katheter 2 am Behandlungsort angekommen ist, wird die Kappe 9 von der relativ gegenüber dem äusseren Katheterschaft 3 sich vorschiebenden Gefässstütze 5 durchstossen und damit geöffnet. Schlitze 10 in der Kappe 9 können diesen Vorgang erleichtern. In Fig. 3 ist die Kappe 9 in ganz geöffnetem Zustand dargestellt, in Fig. 2 ist die in diesem Fall elastische Kappe 9 wieder teilweise geschlossen, da der durchtretende Durchmesser, der Innenkatheter 4, kleiner ist als der grösste durchtretende Durchmesser.

Wenn die Gefässstütze 5 wieder ganz in den äusseren Katheterschaft 3 zurückgezogen ist, verschliesst sich die Kappe 9 wieder, falls es sich um eine elastische Kappe 9 handelt. Da beim Zurückziehen des Katheters 2 aus dem Gefäss 1 nicht mehr die Gefahr besteht, dass die Gefässwand durch den Rand des Katheterschafts 3 verletzt wird, kann die Kappe 9 auch so ausgebildet sein, dass sie sich nicht mehr selbsttätig schliesst nach Gebrauch der Gefässstütze 5, sondern dass die Kappe 9 in der jeweils zuletzt eingenommenen Stellung verbleibt.

## Patentansprüche

1. Katheter mit einer ***selbst expandierenden*** zylindrischen Gefässstütze (5) aus einem durchlässigen Netz sich überkreuzender steifer Fäden (6), wobei die Gefässstütze (5) bei ihrem Einsatz sich aus einem gespannten Zustand mit kleinem Umfang durch ihre radiale Elastizität aus eigener Kraft aufweitet in einen die Gefässwand stützenden entspannten Zustand mit über seine Länge gleichbleibendem grossem Umfang, mit einem ***verschieblichen*** schlauchförmigen äusseren Katheterschaft (3), der an seinem distalen Ende die gespannte Gefässstütze in sich aufnimmt und aus dem die Gefässstütze (5) zu ihrem Einsatz freigegeben werden kann, mit einem verschieblichen Innenkatheter (4) im Innern des schlauchförmigen äusseren Katheterschafts (3), der die Gefässstütze (5) an ihrem proximalen Ende axial abstützt, wobei zur Freigabe der Gefässstütze (5) der äussere Katheterschaft (3) gegenüber dem Innenkatheter (4) zurückgezogen wird, ***wobei*** die Gefässstütze (5) an ihrem proximalen Ende eingespannt so gesichert ist, dass sie einen mit der Gefässstütze (5) sich selbst öffnenden durchlässigen Netzkegel (7) bildet, dessen Radius langsam zum Radius der entspannten Gefässstütze (5) ansteigt, **wobei** die Gefässstütze (5) mit Hilfe des Netzkegels (7) fest am Innenkatheter (4) verankert ist **und *wobei am proximalen Ende des Netzkegels (7) die Gefässstütze (5) auf den Aussendurchmesser des Innenkatheters (4) zusammengezogen ist, dadurch gekennzeichnet, dass die Gefässstütze (5) am proximalen Ende des Netzkegels (7) durch eine Schicht Bindemittel mit dem Innenkatheter (4) verbunden ist.***

2. Katheter nach Anspruch 1, dadurch gekennzeichnet, dass der Innenkatheter (4) schlauchförmig ausgebildet ist und innerhalb der Gefässstütze (5) endet.

3. Katheter nach Anspruch ***1 oder 2***, dadurch gekennzeichnet, dass der äussere Katheterschaft (3) mit einer Schutzkappe (10) versehen ist, die von der Gefässstütze (5) durchstossen werden kann.

4. Verfahren zur Herstellung eines Katheters nach Anspruch 1, dadurch gekennzeichnet, dass ***das proximale Ende*** des Netzkegels (7) der Gefässstütze (5) durch einen Schrumpfschlauch auf dem Aussendurchmesser des Innenkatheters (4) zusammengehalten wird während der Netzkegel (7) mit dem Innenkatheter (4) verbunden wird.

## Claims

1. Catheter with a self-expanding cylindrical vessel support (5) consisting of a permeable mesh of crossing rigid filaments (6), wherein the vessel support (5) expands during use from a tensioned state with a small circumference into a relaxed state supporting the vessel wall with a large circumference which is uniform over its length, due to its intrinsic force owing to its radial elasticity, with a displaceable tubular outer catheter shank (3) which receives the tensioned vessel support in itself at its distal end and from which the vessel support (5) can be released for use thereof, with a displaceable inner catheter (4) in the interior of the tubular outer catheter shank (3) which axially supports the vessel support (5) at its proximal end, wherein the outer catheter shank (3) is retracted relative to the inner catheter (4) to release the vessel support (5), wherein the vessel support (5) is fixed at its proximal end and secured such that it forms a permeable mesh cone (7) which opens itself with the vessel support (5) and of which the radius slowly increases to the radius of the relaxed vessel support (5), wherein the vessel support (5) is rigidly secured on the inner catheter (4) by means of the mesh cone (7) and wherein the vessel support (5) is constricted to the external diameter of the inner catheter (4) at the proximal end of the mesh cone (7), characterized in that the vessel support (5) is connected to the inner catheter (4) at the proximal end of the mesh cone (7) by a layer of binder.

2. Catheter according to Claim 1, characterized in that the inner catheter (4) is tubular in design and ends inside the vessel support (5).

3. Catheter according to Claim 1 or 2, characterized in that the outer catheter shank (3) is provided with a protective cap (10) which can be perforated by the vessel support (5).

4. Method of producing a catheter according to Claim 1, characterized in that the proximal end of the mesh cone (7) of the vessel support (5) is held together on the external diameter of the inner catheter (4) by a shrink tube while the mesh cone (7) is connected to the inner catheter (4).

## Revendications

1. Cathéter comportant un renfort vasculaire (5) cylindrique, auto-dilatable, réalisé en un réseau perméable de fils (6) rigides et entrecroisés, ledit renfort vasculaire (5) se dilatant, lors de son introduction, sous sa propre force de par son élasticité radiale, et passant d'un état tendu à périmètre étroit à un état détendu renforçant la paroi vasculaire à périmètre large et constant sur toute sa longueur, comportant une tige (3) de cathéter extérieure, de forme tubulaire, coulissable, qui reçoit dans son extrémité distale le renfort vasculaire tendu et de laquelle ledit renfort vasculaire (5) peut être dégagé en vue de son utilisation, comportant un cathéter intérieur (4) coulissable à l'intérieur de la tige extérieure (3) de forme tubulaire, lequel cathéter (4) renforçant axialement le renfort (5) à son extrémité proximale, la tige extérieure (3) étant soumise à un mouvement de retrait par rapport au cathéter intérieur (4), pour dégager le renfort vasculaire (5), mouvement pendant lequel le renfort vasculaire (5), serré et engagé sur son extrémité proximale, est fixé de telle sorte qu'il forme un cône réticulé (7) perméable qui s'ouvre avec le renfort (5), cône (7) dont le rayon croît lentement jusqu'à atteindre le rayon du renfort (5) à l'état détendu, ledit renfort vasculaire (5) étant ancré de manière fixe, à l'aide de ce cône réticulé (7), sur le cathéter intérieur (4), tandis qu'à l'extrémité proximale du cône réticulé (7) le renfort vasculaire (5) se rétracte pour atteindre le diamètre extérieur du cathéter intérieur (4), caractérisé en ce qu'à l'extrémité proximale du cône réticulé (7) le renfort vasculaire (5) est relié par une couche d'un produit adhésif au cathéter intérieur (4).

2. Cathéter selon la revendication 1, caractérisé en ce que le cathéter intérieur (4) est réalisé en forme de tuyau flexible et en ce qu'il s'achève à l'intérieur du renfort vasculaire (5).

3. Cathéter selon la revendication 1 ou 2, caractérisé en ce que la tige (3) extérieure du cathéter est munie d'un capuchon de protection (10) qui peut être transpercé par le renfort vasculaire (5).

4. Procédé pour la fabrication d'un cathéter selon la revendication 1, caractérisé en ce que l'extrémité proximale du cône réticulé (7) du renfort vasculaire (5) est maintenue sur le diamètre extérieur du cathéter intérieur (4) au moyen d'un flexible rétractable pendant l'opération d'assemblage entre le cône réticulé (7) et le cathéter intérieur (4).
